# EUROPÄISCHE PATENTSCHRIFT

(11) **EP 4 107 421 B1**
(45) Veröffentlichungstag und Bekanntmachung des Hinweises auf die Patenterteilung: **26.06.2024**
(21) Anmeldenummer: 21708156.1
(22) Anmeldetag: 18.02.2021
(51) Int. Cl.: G01N 33/00

(54) **ANALYSEVERFAHREN FÜR GASE IN EINEM DRUCKGASSPEICHER**
ANALYSIS METHOD FOR GASES IN A COMPRESSED-GAS TANK
PROCÉDÉ D'ANALYSE POUR DES GAZ DANS UN RÉSERVOIR DE GAZ COMPRIMÉ

(30) Priorität: 19.02.2020 DE 102020001077
(43) Veröffentlichungstag der Anmeldung: 28.12.2022
(73) Patentinhaber: Bauer Kompressoren GmbH, 81477 München (DE)
(72) Erfinder: HUBER, Johannes, 86316 Friedberg (DE); BURMEISTER, Thomas, 83679 Sachsenkam (DE); KAMPFL, Robert, 81379 München (DE); FRANTZ, Stefanie, 81373 München (DE)
(74) Vertreter: Weickmann & Weickmann PartmbB
(86) Internationale Anmeldenummer: PCT/EP2021/054032
(87) Internationale Veröffentlichungsnummer: WO 2021/165399

(56) Entgegenhaltungen:
- CN-U- 207 294 167
- DE-T2- 69 837 504
- US-A- 5 810 928
- US-A1- 2019 120 431

## Beschreibung

Die Erfindung bezieht sich auf ein Verfahren zum Betreiben einer Analysevorrichtung für Druckgas in einem Druckgasspeicher, wie einem Atemluftzylinder oder dergleichen. Diese dient zur Bestimmung von Gasbestandteilen, insbesondere CO, CO₂, O₂, VOC, SO₂, NO, NO₂, Helium sowie Feuchte, und hat eine Entnahmeeinrichtung, die mit dem Druckgasspeicher verbunden werden kann.

Die Konzentration sowie die Zusammensetzung und der Druck des sich im Druckgasspeicher befindlichen Druckgases sind unbekannt, es sein denn, dass man für den Druckgasspeicher eine gesonderte Gasmesseinrichtung einsetzt. Die Gasmesseinrichtungen und insbesondere solche, die auch die Feuchte beispielsweise mittels eines Taupunktsensors erfassen, sind in der Anschaffung und Wartung relativ teuer.

Weiterhin ist es aus dem Stand der Technik bekannt, beispielsweise aus der DE 10 2018 008 636 A1, die Luftqualität eines Kompressors zum Füllen eines solchen Druckgasspeichers permanent zu messen, wobei es sich hierbei sowohl um einen tragbaren Druckgasspeicher, wie beispielsweise einen Atemluftzylinder oder ähnliches, oder auch um einen fest installierten Druckgasspeicher handeln kann. Hierbei besteht bei der Überwachung eines Kompressors der Vorteil, dass durch den permanenten Strom an Gas durch die entsprechende Messvorrichtung gleichbleibende Bedingungen hierin herrschen und sich durch die unter Druck stehenden Rohrleitungen weniger Feuchte im System ansammelt.

Weiterhin soll Bezug genommen sein auf die US 5 810 928 A, welche ein gattungsgemäßes Verfahren lehrt, sowie auf die US 2019/120 431 A1, die DE 69 837 504 T2 und die CN 207 294 167 U.

US 2019/120 431 A1 offenbart ein Verfahren zum Befüllen von Druckgasbehältern, wobei eine Analysevorrichtung das von einem Kompressor zum Füllen des Druckgasbehälters abgegebene Gas auf unerwünschte Gasbestandteile analysiert.

Die Erfindung zielt daher darauf ab, unter Überwindung der zuvor geschilderten Schwierigkeiten eine kostengünstigere und zuverlässige Analysevorrichtung der gattungsgemäßen Art bereitzustellen, die zeitnah, schnell und zuverlässig die Gasbestandteile, den Druck und die Feuchte sowohl aus einem Kompressor wie auch aus einem Druckbehälter/Speicher mittels einer einzelnen Gasmess- und Auswerteeinrichtung erfassen kann. Eine Herausforderung kann hierbei darin bestehen, nicht nur den entsprechenden Sensor sondern auch sämtliche Zuleitungen zu der vorgetrockneten Sensorik mit möglichst wenig Feuchte zu belasten, um eine möglichst kurze Spülzeit zu erreichen.

Nach der Erfindung wird hierzu eine Analysevorrichtung für Druckgase in einem Druckgasspeicher, wie einem Atemluftzylinder oder dergleichen, zur Bestimmung von Gasbestandteilen, insbesondere CO, CO₂, O₂, VOC, SO₂, NO, NO₂, Helium sowie Feuchte mit einer Entnahmevorrichtung bereitgestellt, die mit dem Druckgasspeicher verbunden werden kann. Diese Analysevorrichtung zeichnet sich dadurch aus, dass die Entnahmeeinrichtung ein unter Druck des zu analysierenden Druckgases im Druckgasspeicher stehendes Verbindungselement umfasst, welches eine Gasaufbereitungseinrichtung mit einer Gasmess- und Auswerteeinrichtung zum Bestimmen der gewünschten Gasbestandteile anschließbar ist, wobei ggf. das Verbindungselement ein Trockenmittel möglichst im atmosphärischen Bereich umfasst, dass das Verbindungselement, auch im nicht mit dem Druckgasspeicher verbundenen Zustand vorzugsweise mittels des Absperrventils, unter Druck steht, und die Gasaufbereitungseinrichtung und die Gasmess- und Auswerteeinrichtung ferner ebenfalls dazu eingerichtet sind, die von einem Kompressor zum Füllen eines Druckgasspeichers abgegebenen Druckgase zu überwachen und zu analysieren.

Das Trockenmittel kann dazu dienen, im Stillstand die Vortrocknung der atmosphärisch beaufschlagten Bauteile trocken zu halten. Somit verkürzt sich die Wartezeit wesentlich bis eine aussagekräftige Messung möglich ist.

Bei der Analysevorrichtung nach der Erfindung wird über ein Verbindungselement in der Entnahmeeinrichtung die Gaseinrichtung mit einer Auswerteeinrichtung zur Bestimmung der erwünschten Gasbestandteile genutzt, die bereits vorhanden ist, und die vom Kompressor abgegebenen Gase analysiert. Somit wird bei der Erfindung die Gasmesseinrichtung mit einer Auswerteeinrichtung mitgenutzt, welche in der Kompressoranlage vorhanden ist. Hierdurch lassen sich die Anschaffungs- und Wartungskosten reduzieren.

Weitere vorteilhafte Ausgestaltungen der Analysevorrichtung gemäß der Erfindung sind in Unteransprüchen wiedergegeben.

Insbesondere ist es bei der Analysevorrichtung nach der Erfindung wesentlich, dass das Verbindungselement und alle mit dem Druckgas in Berührung kommenden Bereiche feuchtigkeitsabweisende Eigenschaften haben, so dass möglichst zeitnah und möglichst ohne negativen Einfluss der atmosphärisch vorhandenen Feuchte die gewünschten Werte erfasst werden können. Auch ist es zweckmäßig, für das Verbindungselement und die dazugehörigen Teile geeignete Materialien einzusetzen, welche feuchtigkeitsabweisende Eigenschaften haben. Hierbei handelt es sich beispielweise um Edelstahl oder dergleichen. Vorzugsweise hat bei einem Verbindungselement in Form eines Verbindungsschlauches dieser eine PTFE oder FEP-Seele.

Eine solche Gasmesseinrichtung enthält vorzugsweise einen Feuchtesensor, beispielsweise einen Taupunktsensor, welcher relativ teuer und empfindlich ist, und dieser Sensor der Gasmesseinrichtung erfasst bei der Erfindung zugleich auch die Gasanalyse vom Druckgas im Druckgasspeicher.

Um Fälschungen oder Bedienfehler zu vermeiden, umfasst der Druckspeicher einen die Seriennummer identifizierenden RFID-Chip oder einen anderen eindeutig identifizierenden, einlesbaren Code CQR, Barcode oder dergleichen. Dieser lässt sich mittels eines Lesegeräts an der Entnahmeeinrichtung auslesen, und vorzugsweise wird er zu einer Cloud-Speichereinrichtung oder einem anderen Datenspeichermedium, wie USB-Stick oder dergleichen übertragen.

Vorzugsweise hat die Auswerteeinrichtung der Gasmesseinrichtung eine solche Auslegung, dass das Signal betreffend die Gasbestandteile ebenfalls an die Cloud-Speichereinrichtung oder ein anderes Datenspeichermedium, insbesondere mittels Fernübertragung übertragen wird. Somit lässt sich eine Speicherung der wichtigsten Informationen bezüglich der Gaszusammensetzung unter jeweiliger Zuordnung zu einem bestimmten Druckgasspeicher speichern, welche ebenfalls aus der Cloud-Speichereinrichtung oder einem anderen Speichermedium wieder ausgelesen werden können, um die Mess- und Analysewerte zu dokumentieren.

In einer Ausführungsform der Analysevorrichtung können zur Verbindung mit dem Kompressor und dem Druckgasspeicher ein erstes und ein zweites steuerbares Ventil vorgesehen sein, wobei wenigstens eines der steuerbaren Ventile vorzugsweise durch ein Magnetventil gebildet sein kann.

Während es prinzipiell möglich wäre, die steuerbaren Ventile im Hochdruckbereich vorzusehen, da beispielsweise Hochdruck-Magnetventile verfügbar sind, die in Druckbereichen bis 550bar arbeiten, so kann doch ebenfalls den beiden Ventile jeweils ein Druckminderer vorgeschaltet sein. In einer solchen Ausführungsform kann dann jeder der beiden Hochdruckbereiche von Kompressor bzw. Druckgasspeicher über eine eigene Gasentnahmeeinheit, umfassend einen Druckminderer, eine Düse, ein Sicherheitsventil und ein steuerbares Ventil, insbesondere ein Magnetventil, mit der Gasaufbereitungseinrichtung und damit der Gasmess- und Auswerteeinrichtung verbunden sein.

Weiterhin kann die Analysevorrichtung ferner eine Steuerungslogik umfassen, welche dazu eingerichtet ist, dafür zu sorgen, dass stets lediglich eines der steuerbaren Ventile geöffnet sein kann, da sich ansonsten die beiden Analysegasströme vermischen können, was zu Fehlmessungen führen könnte.

In bevorzugter Weise kann die Analysevorrichtung in Kompressor integriert sein, welcher dann dementsprechend eine solche Analysevorrichtung als fest verbaute Komponente umfasst. Insbesondere kann die Analysevorrichtung hierbei derart in dem Kompressor integriert sein, dass sie während eines Betriebs davon eine permanente Überwachung der ausgegebenen Druckgase durchführt.

Wie angesprochen betrifft die vorliegende Erfindung ein Verfahren zum Betreiben einer Analysevorrichtung, wobei in einem ersten Betriebsmodus der Analysevorrichtung die Gasmess- und Auswerteeinrichtung die von einem Kompressor zum Füllen eines Druckgasspeichers abgegebenen Druckgase überwacht und analysiert, während in einem zweiten Betriebsmodus der Analysevorrichtung die Gasmess- und Auswerteeinrichtung von dem Druckgasspeicher abgegebenen Druckgase überwacht und analysiert.

Hierbei kann ein Umschalten zwischen dem ersten Betriebsmodus und dem zweiten Betriebsmodus durch ein manuelles Umschalten erfolgen und/oder es kann bei dem Umschalten wenigstens eines der folgenden Ereignisse ausgelöst werden, um Sicherheitslücken bei der Überwachung des Kompressors zu vermeiden, insbesondere wenn von einer Messung des Kompressors auf eine Messung des Druckgasspeichers umgeschaltet wird:
- der Kompressor wird über einen Alarmkontakt abgeschaltet bzw. am Starten gehindert, hierzu muss eine betriebsmäßige Kopplung zwischen der Steuerung des Kompressors und der Gasaufbereitungseinrichtung vorliegen;
- ein Spülventil wird geöffnet, so dass von dem Kompressor bereitgestelltes Druckgas entweicht und nicht in den Druckgasspeicher gefüllt wird, hierzu muss ein entsprechendes Spülventil für den Kompressor bereitgestellt sein;
- der momentane Betriebsmodus wird signalisiert, so dass kein unbeabsichtigter Betrieb des Kompressors möglich ist, hierzu ist eine entsprechend eingerichtete und gekoppelte Anzeige- oder Signalvorrichtung notwendig.

Weitere Einzelheiten, Merkmale und Vorteile der Erfindung ergeben sich der nachstehenden Beschreibung von bevorzugten Ausführungsformen unter Bezugnahme auf die beigefügte Zeichnung.

Darin zeigt:
Fig. 1 eine schematische Ansicht einer bevorzugten Ausführungsform nach der Erfindung, und zwar einerseits mit einem Druckgasspeicher-Verbindungselement und andererseits im Grundzustand und abgekoppelt vom Druckgasspeicher;
Fig.2 eine Seitenansicht und Schnittansicht des an den Druckgasspeicher anzuschließenden Verbindungselements;
Fig. 3 schematisch eine Gesamtansicht einer Kompressoranlage mit einer Messeinrichtung und einer Auswerteeinrichtung, die bei der Erfindung auch für die Analyse und Bestimmung der Bestandteile des Druckgases in dem Druckgasspeicher genutzt wird;

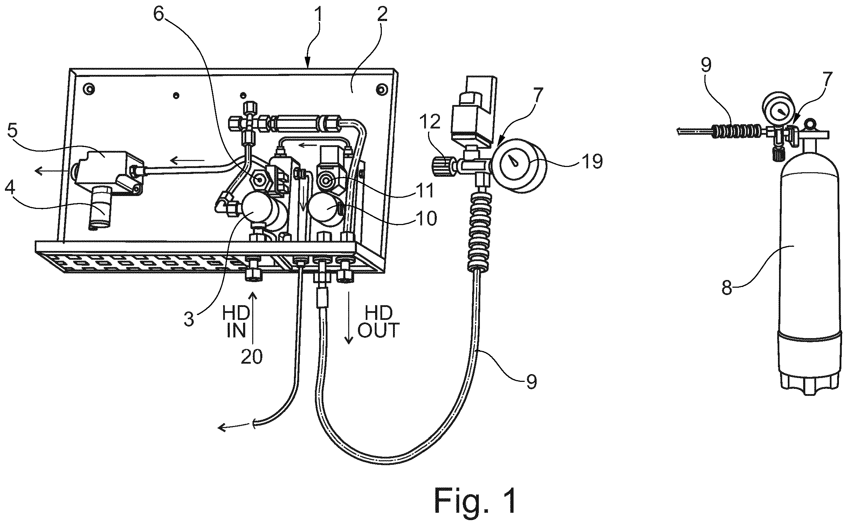

Im linken Teil von Fig. 1 wird eine insgesamt mit 1 bezeichnete Analysevorrichtung gezeigt. Dieser Analysevorrichtung 1 umfasst eine Gasaufbereitungseinrichtung, welche insgesamt mit 2 bezeichnet ist. Wie mit HD-IN und HD-OUT angedeutet ist, handelt es sich hierbei um den Eingang des vom Kompressor beispielsweise gelieferten Hochdruckgases, welches nach Durchgang durch die Gasaufbereitungseinrichtung 2 über HD-OUT austritt. Mit 3 ist ein Druckminderer für die vom Hochdruckkompressor kommenden Druckgase bezeichnet. Ferner umfasst die Gasaufbereitungseinrichtung 2 einen Taupunktsensor 4, eine Feuchtemesseinheit 5, welche ein nicht näher dargestelltes Trockenmittel und einen Durchflusssensor enthält. Dem Druckminderer 3 nachgeschaltet ist ein Magnetventil 6.

Ebenfalls in dieser Fig. ist eine Entnahmeeinrichtung 7 gezeigt, welche mit einem Druckgasspeicher 8 in Form eines Atemluftzylinders beispielsweise über ein Verbindungselement 9 verbunden werden kann. Im linken Teil von Fig. 1 ist die Entnahmeeinrichtung 7 mit dem Verbindungselement 9 in Form eines Verbindungsschlauches von dem Druckgasspeicher 8 abgekoppelt dargestellt. Das Verbindungselement 9 stellt eine Anschlussverbindung mit der Gasaufbereitungseinrichtung 2 her, und über einen Druckminderer und einem Magnetventil 6 wird eine kommunizierende Leitungsverbindung mit dem Taupunktsensor 4 der Feuchtemesseinheit 5 hergestellt.

Bei der erfindungsgemäßen Lösung wird bei der Analysevorrichtung 1 die Gasaufbereitungseinrichtung genutzt, welche für den hier nicht näher dargestellten Kompressor vorgesehen ist.

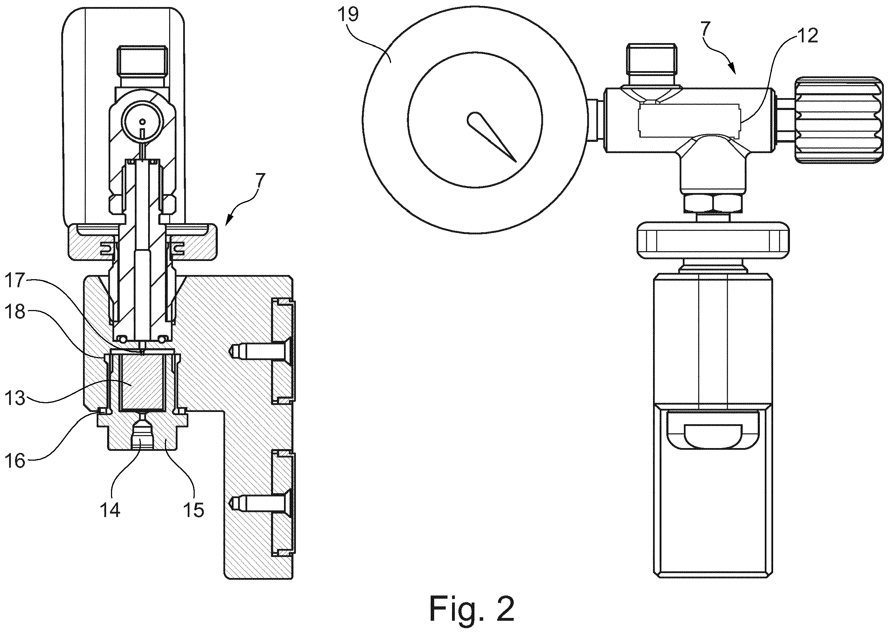

Die Entnahmeeinrichtung wird nachstehend an Hand von den Darstellungen in Fig. 2 näher erläutert.

Wichtig ist, dass das Verbindungselement 9 auch im nicht mit dem Druckgasspeicher verbundenen Zustand unter Druck steht, und zwar vorzugsweise mittels eines Absperrventils.

Aus den beiden Ansichten von Fig. 2 ist zu ersehen, dass die Entnahmeeinrichtung 7 ein Trockenmittel (Trockenkapsel) 13, ein Rückschlagventil 14, eine Verschlussschraube 15, eine metallische Dichtung 16, eine Düse 17 und ein Elastomerdichtung aufweist. Das Absperrventil 12 ist dort ebenfalls als doppelseitiges Absperrventil dargestellt. Auch ist der Entnahmeeinrichtung 7 ein Manometer 19 zugeordnet. Die Entnahmeeinrichtung 7 ist entsprechend der Schnittdarstellung in Fig. 2 derart ausgelegt, dass das Verbindungselement 9 hinsichtlich des inneren Durchganges feuchtigkeitsabweisend ist, sodass mit Hilfe der Gasaufbereitungseinrichtung 2 (Fig. 2) zeitnahe und zuverlässige Messergebnisse erfasst werden können, welche weitgehend frei von Einflüssen durch die atmosphärische Feuchte sind.

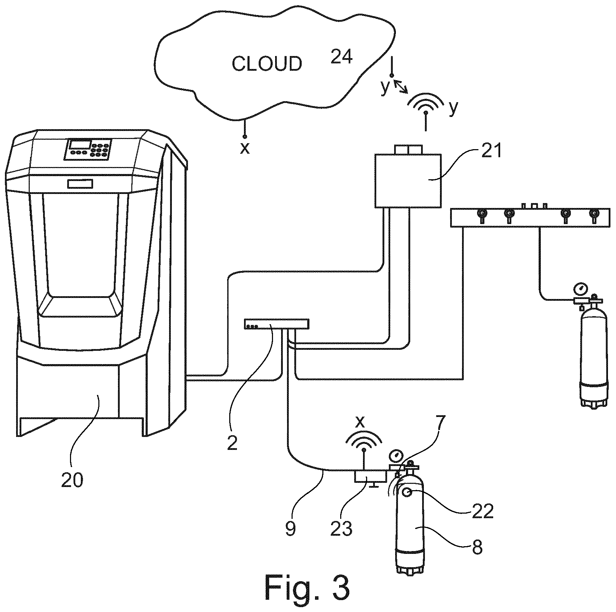

Fig. 3 zeigt schematisch eine Gesamtansicht des erfindungsgemäßen Konzepts. Mit einem Kompressor 20 ist eine Gasaufbereitungseinrichtung 2 verbunden, welche in Fig. 1 beispielsweise hinsichtlich des näheren Aufbaus verdeutlicht ist. Diese Gasaufbereitungseinrichtung 2 ist mit einer Gasmess- und Auswerteeinrichtung 21 verbunden, welche die mit Hilfe der Gasmesssensorik erfassten Messdaten näher auswertet.

Wie aus der schematischen Darstellung von Fig. 3 zu ersehen ist, hat der Druckgasspeicher 8 in Form eines Atemluftzylinders einen RFID-Chip, welcher beispielsweise die eindeutige Seriennummer des Druckgasspeichers 8 enthält. Mit Hilfe eines Lesegeräts 23 werden die Informationen des RFID-Chips 22 ausgelesen, und wie schematisch mit X angedeutet über eine Fernübertragung zu einer Cloud-Speichereinrichtung 24 übertragen. Alternativ könnte ein anderer eindeutig den Druckgasspeicher 8 identifizierender einlesbarer Code, CQR, Barcode oder dergleichen, gewählt werden Die Gasmess- und Auswerteeinrichtung 21 übermittelt per Fernübertragung, welche schematisch mit Y angedeutet ist, die ausgewerteten Daten ebenfalls zu der Cloud-Speichereinrichtung 24. Alternativ können die Daten auch auf ein anderes externes Speichermedium übertragen werden.

Somit sind in der Cloud-Speichereinrichtung 24 beispielsweise die entsprechende Ergebnisse der Gasmess- und Auswerteeinrichtung 21 eindeutig dem Druckgasspeicher 8 zugeordnet, und zwar über den RFID-Chip, sodass die erfassten Daten in der Cloud-Speichereinrichtung 24 oder einem anderen externen Speichermedium zusammengeführt werden.

### Bezugszeichenliste

- 1: Analysevorrichtung insgesamt
- 2: Gasaufbereitungseinrichtung
- 3: Druckminderer
- 4: Feuchtesensor, beispielsweise Taupunktsensor
- 5: Feuchtemesseinheit
- 6: Magnetventil
- 7: Entnahmeeinrichtung
- 8: Druckgasspeicher in Form eines Atemluftzylinders
- 9: Verbindungselement, vorzugsweise in Form eines Verbindungsschlauchs
- 10: Druckminderer
- 11: Magnetventil
- 12: Absperrventil
- 13: Trockenmittel
- 14: Rückschlagventil
- 15: Verschlussschraube
- 16: metallische Dichtung
- 17: Düse
- 18: Elastomerdichtung
- 19: Manometer
- 20: Kompressor
- 21: Gasmess- und Auswerteeinrichtung
- 22: RFID-Chip
- 23: Lesegerät
- 24: Cloud-Speichereinrichtung

## Patentansprüche

1. Verfahren zum Betreiben einer Analysevorrichtung für Druckgase in einem Druckgasspeicher, wie einem Atemluftzylinder oder dergleichen, zur Bestimmung von Gasbestandteilen, insbesondere CO, CO₂, O₂, VOC, SO₂, NO, NO₂, Helium sowie Feuchte, mit einer Entnahmeeinrichtung (7), die mit dem Druckgasspeicher (8) verbindbar ist, wobei die Entnahmeeinrichtung (7) ein unter Druck des zu analysierenden Druckgases im Druckgasspeicher (8) stehendes Verbindungselement (9) umfasst, welches an eine Gasaufbereitungseinrichtung (2) mit einer Gasmess- und Auswerteeinrichtung (21) zur Bestimmung der gewünschten Gasbestandteile angeschlossen ist, das Verbindungselement (9) auch im nicht mit dem Druckgasspeicher (8) verbundenen Zustand, vorzugsweise mittels eines Absperrventils (12), unter Druck steht, und die Gasaufbereitungseinrichtung (2) und die Gasmess- und Auswerteeinrichtung (21) ferner ebenfalls dazu eingerichtet sind, die von einem Kompressor (20) zum Füllen eines Druckgasspeichers abgegebenen Druckgase zu überwachen und zu analysieren, **dadurch gekennzeichnet, dass** in einem ersten Betriebsmodus der Analysevorrichtung die Gasmess- und Auswerteeinrichtung (21) die von einem Kompressor (20) zum Füllen eines Druckgasspeichers abgegebenen Druckgase analysiert, während in einem zweiten Betriebsmodus der Analysevorrichtung die Gasmess- und Auswerteeinrichtung (21) von dem Druckgasspeicher (8) abgegebenen Druckgase überwacht und analysiert.

2. Verfahren nach Anspruch 1, **dadurch gekennzeichnet, dass** das Verbindungselement (9) ein Trockenmittel (13) möglichst im atmosphärischen Bereich zum Vortrocknen der atmosphärisch beaufschlagten Bauteile im Stillstand umfasst.

3. Verfahren nach Anspruch 1 oder 2, **dadurch gekennzeichnet, dass** das Verbindungselement (9) und alle mit dem Druckgas in Berührung kommenden Bereiche feuchtigkeitsabweisende Eigenschaften haben.

4. Verfahren nach einem der vorhergehenden Ansprüche , **dadurch gekennzeichnet,**
**dass** das Verbindungselement (9) in Form eines Verbindungsschlauchs ausgebildet ist.

5. Verfahren nach Anspruch 4, **dadurch gekennzeichnet, dass** der Verbindungsschlauch eine PTFE oder FEP-Seele zur Feuchtigkeitsabweisung besitzt.

6. Verfahren nach einem der Ansprüche 1 bis 5, **dadurch gekennzeichnet, dass** ein Feuchtesensor (4) der Gasaufbereitungseinrichtung (2) zugleich auch die Gasanalyse vom Druckgas im Druckgasspeicher (8) erfasst.

7. Verfahren nach einem der Ansprüche 1 bis 6, **dadurch gekennzeichnet, dass** der Druckgasspeicher (8) einen die Seriennummer identifizierenden RFID-Chip (22) umfasst, welcher mittels eines Lesegeräts (23) an der Entnahmeeinrichtung (7) lesbar und zu einer Cloud-Speichereinrichtung (24) übertragbar ist.

8. Verfahren nach Anspruch 7, **dadurch gekennzeichnet, dass** die Gasmess- und Auswerteeinrichtung (21) der Gasaufbereitungseinrichtung (2) Signale betreffend die Gasbestandteile an die Cloud-Speichereinrichtung (24) mittels Fernübertragung überträgt.

9. Verfahren nach einem der vorangehenden Ansprüche, **dadurch gekennzeichnet, dass** dem Trockenmittel (13) eine Düse (17) vorgeschaltet ist, welche einen schnellen Eintrag der Umgebungsfeuchte in den Messbereich verhindert.

10. Verfahren nach einem der vorangehenden Ansprüche, **dadurch gekennzeichnet, dass** das Trockenmittel (13) mittels einer Verschlussschraube (15) zum Auswechseln des gesättigten Trockenmittels demontierbar angeordnet ist.

11. Verfahren nach einem der vorhergehenden Ansprüche, **dadurch gekennzeichnet, dass** zur Verbindung mit dem Kompressor (20) und dem Druckgasspeicher (8) ein erstes (6) und ein zweites (11) steuerbares Ventil vorgesehen sind, wobei wenigstens eines der steuerbaren Ventile (6, 11) vorzugsweise durch ein Magnetventil gebildet ist.

12. Verfahren nach Anspruch 11, **dadurch gekennzeichnet, dass** den beiden steuerbaren Ventilen (6, 11) jeweils ein Druckminderer (3, 10) vorgeschaltet ist.

13. Verfahren nach einem der Ansprüche 11 und 12, **dadurch gekennzeichnet, dass** sie ferner eine Steuerungslogik umfasst, welche dazu eingerichtet ist, dafür zu sorgen, dass stets lediglich eines der steuerbaren Ventile (6, 11) geöffnet sein kann.

14. Verfahren nach einem der vorhergehenden Ansprüche, wobei die eine Analysevorrichtung als fest verbaute Komponente in einem Kompressor umfasst ist, wobei vorzugsweise die Analysevorrichtung dazu eingerichtet und derart in dem Kompressor integriert ist, während eines Betriebs davon eine permanente Überwachung der ausgegebenen Druckgase durchzuführen.

15. Verfahren nach einem der vorhergehenden Ansprüche, **dadurch gekennzeichnet, dass** ein Umschalten zwischen dem ersten Betriebsmodus und dem zweiten Betriebsmodus durch ein manuelles Umschalten erfolgt und/oder bei dem Umschalten wenigstens eines der folgenden Ereignisse ausgelöst wird:
- der Kompressor (20) wird über einen Alarmkontakt abgeschaltet bzw. am Starten gehindert;
- ein Spülventil wird geöffnet, so dass von dem Kompressor (20) bereitgestelltes Druckgas entweicht und nicht in den Druckgasspeicher (8) gefüllt wird;
- der momentane Betriebsmodus wird signalisiert, so dass kein unbeabsichtigter Betrieb des Kompressors (20) möglich ist.

## Claims

1. Method for operating an analysis apparatus for compressed gases in a compressed-gas tank, such as a breathing-air cylinder or the like, for determining gas components, in particular CO, CO₂, O₂, VOC, SO₂, NO, NO₂, helium and moisture, having a removal device (7) which can be connected to the compressed-gas tank (8), wherein the removal device (7) comprises a connection element (9) which is under the pressure of the compressed gas to be analysed in the compressed-gas tank (8) and can be coupled to a gas treatment device (2) having a gas measuring and evaluating device (21) for determining the desired gas components, the connection element (9) is also pressurised when not connected to the compressed-gas tank (8), preferably by means of a shut-off valve (12), and the gas treatment device (2) and the gas measuring and evaluating device (21) are further also configured to monitor and analyse the compressed gases dispensed by a compressor (20) to fill a compressed-gas tank, **characterised in that** in a first operating mode of the analysis apparatus the gas measuring and evaluating device (21) analyses the compressed gases dispensed by a compressor (20) for filling a compressed-gas tank, while in a second operating mode of the analysis apparatus the gas measuring and evaluating device (21) monitors and analyses compressed gases dispensed by the compressed-gas tank (8).

2. Method according to claim 1, **characterised in that** the connection element (9) comprises a desiccant (13), if possible in the atmospheric region, for pre-drying the components exposed to the atmosphere during standstill.

3. Method according to either claim 1 or claim 2, **characterised in that** the connection element (9) and all regions which come into contact with the compressed gas have moisture-repellent properties.

4. Method according to any of the preceding claims, **characterised in that** the connection element (9) is designed in the form of a connection hose.

5. Method according to claim 4, **characterised in that** the connection hose has a PTFE or FEP core to repel moisture.

6. Method according to any of claims 1 to 5, **characterised in that** a moisture sensor (4) of the gas treatment device (2) at the same time also records the gas analysis of the compressed gas in the compressed-gas tank (8).

7. Method according to any of claims 1 to 6, **characterised in that** the compressed-gas tank (8) comprises an RFID chip (22) identifying the serial number, which RFID chip can be read by means of a reader (23) on the removal device (7) and transmitted to a cloud storage device (24).

8. Method according to claim 7, **characterised in that** the gas measuring and evaluating device (21) of the gas treatment device (2) transmits signals relating to the gas components to the cloud storage device (24) by means of remote transmission.

9. Method according to any of the preceding claims, **characterised in that** a nozzle (17) is connected upstream of the desiccant (13), which nozzle prevents rapid entry of ambient moisture into the measuring region.

10. Method according to any of the preceding claims, **characterised in that** the desiccant (13) is arranged so that it can be removed by means of a locking screw (15) in order to replace the saturated desiccant.

11. Method according to any of the preceding claims, **characterised in that** a first (6) and a second (11) controllable valve are provided for connection to the compressor (20) and the compressed-gas tank (8), wherein at least one of the controllable valves (6, 11) is preferably formed by a solenoid valve.

12. Method according to claim 11, **characterised in that** a pressure reducer (3, 10) is connected upstream of each of the two controllable valves (6, 11).

13. Method according to either claim 11 or claim 12, **characterised in that** it further comprises control logic which is configured to ensure that only one of the controllable valves (6, 11) can be open at all times.

14. Method according to any of the preceding claims, wherein the an analysis apparatus is included as a permanently installed component in a compressor, wherein the analysis apparatus is preferably configured and integrated in the compressor in such a way that, during operation thereof, it carries out constant monitoring of the discharged compressed gases.

15. Method according to any of the preceding claims, **characterised in that** switching between the first operating mode and the second operating mode takes place by manual switching and/or at least one of the following events is triggered during the switching:
- the compressor (20) is switched off or prevented from starting via an alarm contact;
- a rinsing valve is opened so that the compressed gas provided by the compressor (20) escapes and is not filled into the compressed-gas tank (8);
- the current operating mode is signalled so that no unintentional operation of the compressor (20) is possible.

## Revendications

1. Procédé d'exploitation d'un dispositif d'analyse de gaz comprimés dans un réservoir de gaz comprimés, tel qu'un cylindre d'air respirable ou similaire, pour déterminer des composants gazeux, en particulier CO, CO₂, O₂, VOC, SO₂, NO, NO₂, hélium ainsi que l'humidité, comprenant une unité de prélèvement (7) apte à être reliée au réservoir de gaz comprimés (8),
dans lequel l'unité de prélèvement (7) comprend un élément de liaison (9) qui est soumis à la pression du gaz comprimé à analyser dans le réservoir de gaz comprimés (8) et qui peut être raccordé à une unité de traitement de gaz (2) munie d'une unité de mesure et d'évaluation de gaz (21) pour déterminer les composants gazeux souhaités,
l'élément de liaison (9) est soumis à la pression également à l'état non relié au réservoir de gaz comprimés (8), de préférence au moyen d'une vanne d'arrêt (12), et
l'unité de traitement de gaz (2) et l'unité de mesure et d'évaluation de gaz (21) sont en outre également conçues pour surveiller et analyser les gaz comprimés délivrés par un compresseur (20) pour remplir un réservoir de gaz comprimés,
**caractérisé en ce que**, dans un premier mode d'exploitation du dispositif d'analyse, l'unité de mesure et d'évaluation de gaz (21) surveille et analyse les gaz comprimés délivrés par un compresseur (20) pour remplir un réservoir de gaz comprimés, tandis que, dans un deuxième mode d'exploitation du dispositif d'analyse, l'unité de mesure et d'évaluation de gaz (21) surveille et analyse les gaz comprimés délivrés par le réservoir de gaz comprimés (8).

2. Procédé selon la revendication 1,
**caractérisé en ce que** l'élément de liaison (9) comprend un dessiccateur (13), si possible dans la zone de l'atmosphère, pour présécher les composants soumis à l'atmosphère, à l'arrêt.

3. Procédé selon la revendication 1 ou 2,
**caractérisé en ce que** l'élément de liaison (9) et toutes les zones venant en contact avec le gaz comprimé ont des propriétés de répulsion de l'humidité.

4. Procédé selon l'une des revendications précédentes,
**caractérisé en ce que** l'élément de liaison (9) est réalisé sous la forme d'un tuyau flexible de liaison.

5. Procédé selon la revendication 4,
**caractérisé en ce que** le tuyau flexible de liaison possède une âme en PTFE ou en FEP pour repousser l'humidité.

6. Procédé selon l'une des revendications 1 à 5,
**caractérisé en ce qu'**un capteur d'humidité (4) de l'unité de traitement de gaz (2) détecte en même temps également l'analyse du gaz comprimé dans le réservoir de gaz comprimés (8).

7. Procédé selon l'une des revendications 1 à 6,
**caractérisé en ce que** le réservoir de gaz comprimés (8) comprend une puce RFID (22) identifiant le numéro de série, qui peut être lue au moyen d'un lecteur (23) sur l'unité de prélèvement (7) et être transmise à une unité de stockage en nuage (24).

8. Procédé selon la revendication 7,
**caractérisé en ce que** l'unité de mesure et d'évaluation de gaz (21) de l'unité de traitement de gaz (2) transmet des signaux, concernant les composants gazeux, par télétransmission à l'unité de stockage en nuage (24).

9. Procédé selon l'une des revendications précédentes,
**caractérisé en ce que** le dessiccateur (13) est précédé d'une buse (17) qui empêche une entrée rapide de l'humidité ambiante dans la zone de mesure.

10. Procédé selon l'une des revendications précédentes,
**caractérisé en ce que** le dessiccateur (13) est disposé de manière démontable au moyen d'une vis de fermeture (15) pour le remplacement du dessiccateur saturé.

11. Procédé selon l'une des revendications précédentes,
**caractérisé en ce qu'**une première vanne (6) et une deuxième vanne (11) commandables sont prévues pour être reliées au compresseur (20) et au réservoir de gaz comprimés (8), au moins l'une des vannes commandables (6, 11) étant de préférence formée par une électrovanne.

12. Procédé selon la revendication 11,
**caractérisé en ce qu'**un réducteur de pression (3, 10) respectif est monté en amont de chacune des deux vannes commandables (6, 11).

13. Procédé selon l'une des revendications 11 et 12,
**caractérisé en ce qu'**il comprend en outre une logique de commande conçue pour assurer qu'une seule des vannes commandables (6, 11) puisse être ouverte à la fois.

14. Procédé selon l'une des revendications précédentes,
dans lequel le dispositif d'analyse est prévu en tant que composant monté de manière fixe dans un compresseur, de préférence le dispositif d'analyse étant conçu et intégré dans le compresseur de manière à effectuer, pendant son fonctionnement, une surveillance permanente des gaz comprimés délivrés.

15. Procédé selon l'une des revendications précédentes,
**caractérisé en ce qu'**une commutation entre le premier mode d'exploitation et le deuxième mode d'exploitation est effectuée par une commutation manuelle, et/ou
lors de la commutation, l'un au moins des événements suivants est déclenché :
- le compresseur (20) est arrêté ou empêché de démarrer via un contact d'alarme ;
- une vanne de rinçage est ouverte, de sorte que le gaz comprimé fourni par le compresseur (20) s'échappe et n'est pas chargé dans le réservoir de gaz comprimés (8) ;
- le mode d'exploitation momentané est signalé, de sorte qu'aucun fonctionnement involontaire du compresseur (20) n'est possible.
